# EUROPEAN PATENT APPLICATION

(11) **EP 3 492 475 A1**
(43) Date of publication of application: **05.06.2019**
(21) Application number: 17306689.5
(22) Date of filing: 01.12.2017
(51) Int. Cl.: C07D 519/00, C07D 493/08

(54) **NEW CYCLOADDUCT PRECURSORS OF DIHALOBENZOPHENONES AND PREPARATIONS THEREOF**

(71) Applicant: RHODIA OPERATIONS, 75009 Paris (FR)
(72) Inventor: MULLER, Eric, 69003 LYON (FR)
(74) Representative: Delenne, Marc

(57) **Abstract**

The invention relates to new compounds of formula (I): 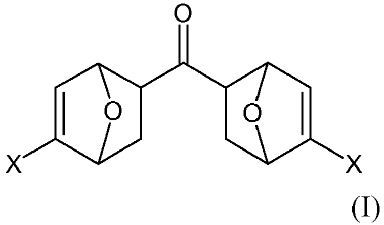 wherein X represents a halogen atom selected from the group consisting of fluorine, chlorine, bromine and iodine, which are useful for the preparation of 4,4'dihalobenzophenones of formula (III): 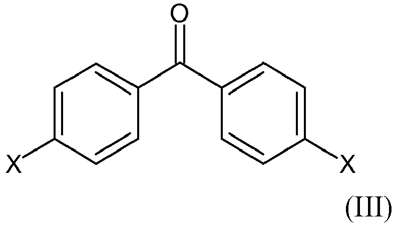 wherein X is as defined above.

## Description

### TECHNICAL FIELD

The present invention pertains, as new and useful chemical compounds, to specific di-(halo-oxanorbornene)ketones, obtainable by cycloaddition between a divinylketone and a halofuran (Diels-Alder reaction) and to their use as precursors for the preparation of dihalobenzophenones.

### BACKGROUND ART

Dihalobenzophenones are valuable chemical compounds which are known to the art. They are mainly prepared by oxidation of the methylene group of a dihalodiphenyl methane to provide the corresponding dihalobenzophenone. Various other methods are known, such as those described in US 4943358, WO 2012/001131, US 5777172 and in Dunlop et al., "The preparation of 4-fluoro- and 4,4'-difluorobenzophenone", J. Am. Chem. Soc., 1933, 55(4), pp. 1665-1666. A non-exhaustive summary of other available processes for the manufacture of 4,4'-difluorobenzophenone may also be found in US 7687668.

4,4'-difluorobenzophenone (4,4'-DFBP) is the central starting material for preparing poly(aryl ether ketone)s (PAEK) which are a well-known class of engineering polymers used in various fields. These are high-performance polymers with constantly growing annual production volumes, so that the volume of 4,4'-DFBP produced worldwide annually is also included in the growth. The most important PAEK are the poly(ether ketone) (PEK) and poly(ether ether ketone) (PEEK), which feature melting points of above 330°C and high chemicals resistance.

The present invention aims at providing a new and efficient process for the preparation of dihalobenzophenones.

Another objective of the present invention is to provide a process for the preparation of dihalobenzophenones involving renewable starting materials.

### SUMMARY OF THE INVENTION

The present invention therefore relates to a compound of formula (I): 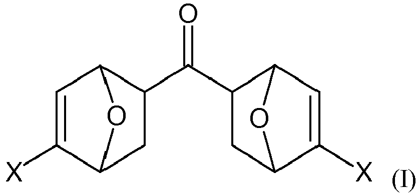 wherein X represents a halogen atom selected from the group consisting of fluorine, chlorine, bromine and iodine.
Preferably, the halogen atom is selected from bromine and chlorine, more preferably chlorine.

Another object of the invention relates to a process for the preparation of a compound of formula (I) as defined above, comprising reacting divinylketone with a halofuran of formula (II): 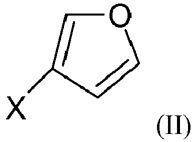 wherein X is as defined above,
and optionally further isolating compound of formula (I).
Preferably, the halofuran is 3-chlorofuran or 3-bromofuran.

According to an embodiment, the molar ratio of the halofuran of formula (II) to divinylketone is of at least 2/1.

The present invention further relates to the use of a compound of formula (I) as defined above, for the preparation of a 4,4'-dihalobenzophenone of formula (III): 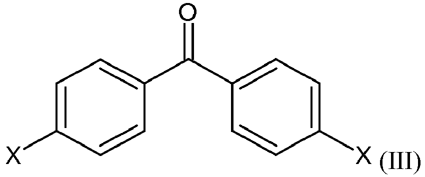 wherein X is as defined above.
Another object of the invention relates to a process for the preparation of a 4,4'-dihalobenzophenone of formula (III): 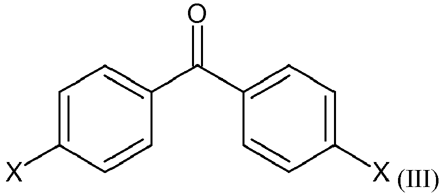 wherein X represents a halogen atom selected from the group consisting of fluorine, chlorine, bromine and iodine,
comprising carrying out the dehydration/aromatization of a compound of formula (I): 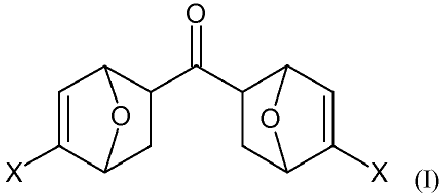 wherein X is as defined above.

According to an embodiment, the dehydration/aromatization is carried out in the presence of an alkali methoxide, preferably sodium methoxide or potassium methoxide, or an alkali hydroxide, preferably sodium hydroxide or potassium hydroxide.

According to an embodiment, the dehydration/aromatization is carried out in the presence of a solvent. The solvent is advantageously chosen from alcohols, sulfoxides and mixtures thereof. It is preferably chosen from C₁-C₆ (especially C₁-C₄) aliphatic alcohols, cyclohexanol, dialkyl sulfoxides wherein each out of the 2 alkyl groups contains from 1 to 6 carbon atoms (especially from 1 to 4 carbon atoms), alkyl phenyl sulfoxides wherein the alkyl group contains from 1 to 6 carbon atoms (especially from 1 to 4 carbon atoms), tetrahydrothiophene 1-oxide and mixtures thereof. Good results were obtained notably when using dimethyl sulfoxide (DMSO) as the solvent.

Another object of the invention relates to a process for the preparation of a 4,4'-dihalobenzophenone of formula (III): 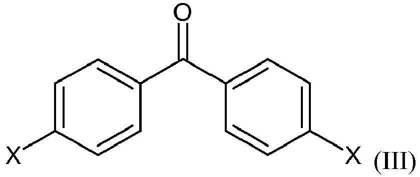 wherein X represents a halogen atom selected from the group consisting of fluorine, chlorine, bromine and iodine,
comprising:
a) providing a compound of formula (I): 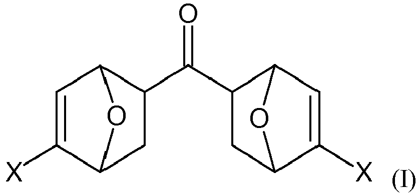 wherein X is as defined above,
b) carrying out the dehydration/aromatization of said compound of formula (I),
c) optionally further isolating compound of formula (III).

According to an embodiment of the invention, compound of formula (I) is prepared by reacting divinylketone with a halofuran of formula (II): 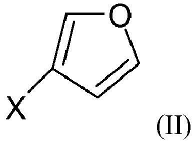 wherein X is as defined above.

As already mentioned, one of the advantages of the invention is to provide a process for the preparation of 4,4'-dihalobenzophenones involving renewable starting materials.

Moreover, it has been surprisingly found that the process for the preparation of compound of formula (I) according to the invention allows satisfactory conversion and selectivity.

### DETAILED DESCRIPTION OF THE INVENTION

The present invention is directed to a compound of formula (I): 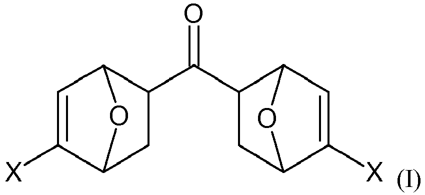 wherein X represents a halogen atom selected from the group consisting of fluorine, chlorine, bromine and iodine.
In a preferred embodiment of the invention, the halogen atom is selected from bromine and chlorine, more preferably chlorine.

Compound of formula (I) may be prepared from divinylketone and a halofuran of formula (II): 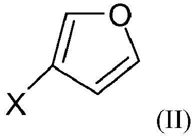 wherein X is as defined above.
Preferably, the halofuran of formula (II) is 3-chlorofuran or 3-bromofuran.

The halofuran of formula (II) can be obtained from furan according to processes known to the person skilled in the art. Document US 2,773,882 describes such a process.
Furan may be prepared by decarbonylation of furfural which is obtained from a renewable resource, see for example US 4,764,627 and US 8,754,245. Suitable renewable resources as well as suitable methods for their conversion into furfural are known to the person skilled in the art.
Alternatively, the halofuran of formula (II) may be prepared by any other conventional chemical reactions, or may be also commercially available products.

Divinylketone can be obtained from formaldehyde and acetone, for example via the Mannich reaction as described in J. Gen. Chem. USSR, Volume 33(5), p. 1512 (1963), from 1,5-dibromopentan-3-one as described in Chem. Eur. J. 2007, 13, pp. 3354-3368, or by oxidation of divinyl carbinol as described in Org. Biomol. Chem., 2010, 8, pp. 751-754.

It has been surprisingly found that a Diels-Alder reaction efficiently occurs between the halofuran of formula (II) and divinylketone resulting in the formation of a double cycloadduct of formula (I) as defined above. Moreover, it has been surprisingly found that the Diels-Alder reaction between the halofuran of formula (II) and divinylketone leads to the formation of compounds of formula (I) with a good conversion, a low amount of by-products and a good selectivity (i.e. para/para).

The present invention therefore also provides a process for the production of a compound of formula (I), comprising reacting divinylketone with a halofuran of formula (II).

The Diels-Alder reaction between the halofuran of formula (II) and divinylketone leads to the formation of two main structural isomers represented below, one of which being the compound of formula (I):

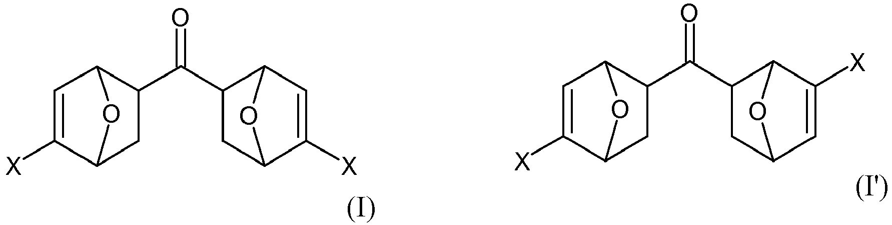

Other isomers might be also obtained in minor amounts, in particular the compound of formula (I"):

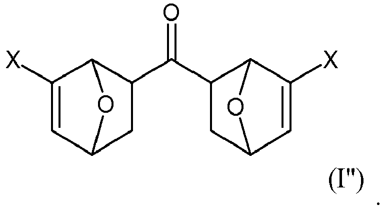

Compounds of formula (I), (I') and (I") can be present as endo- or exo-isomer. In particular, each cycle of the compounds of formula (I), (I') and (I") can be either in exo configuration or endo configuration, also these possible isomers are included within the scope of the present invention.

According to an embodiment of the invention, the molar ratio of compounds of formula (I) to compound of formula (I') is of at least 50/50, preferably at least 70/30, more preferably at least 80/20.

According to an embodiment of the invention, the conversion of the Diels-Alder reaction is of at least 50%, preferably at least 70%, more preferably at least 90%.

The Diels-Alder reaction between the halofuran of formula (II) and divinylketone can be carried out under usual Diels-Alder conditions known to the person skilled in the art.

The Diels-Alder reaction may be conducted with or without a catalyst. The use of catalysts can improve kinetics and selectivity of the Diels-Alder reaction. Known Diels-Alder catalysts may be used and may include Lewis acids, such aluminium chloride, ethylaluminium dichloride, diethylaluminium chloride, trimethyl aluminium, bismuth (III) chloride, bismuth (III) trifluoromethanesulfonate, boron trifluoride, boron triacetate, cerium (III) chloride, copper (I) trifluoromethanesulfonate, copper (II) chloride, hafnium (IV) chloride, iron (II) chloride, iron (II) acetate, iron (III) chloride, iron (III) acetate, lithium perchlorate, lithium trifluoromethanesulfonate, magnesium bromide, magnesium iodide, magnesium chloride, magnesium perchlorate, scandium (III) trifluoromethanesulfonate, tin (IV) chloride, titanium (IV) chloride, titanium (IV) isopropoxide, N-trimethylsilyl-bis(trifluoromethanesulfonyl)imide, trimethylsilyl trifluoromethanesulfonate, ytterbium (III) trifluoromethanesulfonate, zinc chloride, zinc bromide, zinc iodide, zinc acetate and zirconium (IV) chloride, indium (III) chloride, triphenylborane, Bronsted acids, such as inorganic mineral acids, e.g. sulphuric acid, phosphoric acid, nitric acid, hydrobromic acid or hydrochloric acid, and organic acids such as methane sulphonic acid, p-toluenesulphonic acid or carboxylic acids. One of the preferred Diels-Alder catalysts is magnesium iodide.

Alternatively, activated carbon, silica, alumina, silica-alumina, zirconia and zeolites may be used as such or as support for a catalytically active metal or metal compound. Suitable metals or metal compounds include alkali metals, alkaline earth metals, transition metals, noble metals, rare earth metals. The catalysts can be acidic, e.g. by treating supports with phosphoric acid, or by ion exchange of zeolites to render them into their acidic form. Examples of solid catalysts include amorphous silica-alumina, zeolites, preferably zeolites in their H-form, and acidic ion exchange resins. Other suitable catalysts that are liquids or that may be dissolved in the appropriate solvent to yield a homogeneous catalyst environment, include organic and inorganic acids, such as alkane carboxylic acid, arene carboxylic acid, sulphuric acid, phosphoric acid, hydrochloric acid, hydrobromic acid and nitric acid.

The Diels-Alder reaction may be carried out with or without a solvent. Suitable solvents may be selected from pyridine, tertiary amines such as triethylamine and diisopropylethylamine, chloroform, dichloromethane, diethyl ether, perfluorinated alkanes such as perfluorohexane, toluene, water and ionic liquids such as 1-butyl-3-methylimidazolium tetrafluoroborate and 1-butyl-3-methylimidazolium hexafluorophosphate, alone or in a mixture. Preferred solvents are pyridine and tertiary amines, such as triethylamine. In case a catalyst is used, it is preferred to use dichloromethane as solvent or to use no solvent at all.

The Applicant has also surprisingly noticed that, under solvent conditions, certain basic additives such as pyridine and tertiary amines, such as triethylamine, stabilize the reactants which allows an excess of the halofuran of formula (II) to be used in order to increase the conversion of the Diels-Alder reaction. The excess of the halofuran used may be recovered once the reaction is completed.

The reaction may be carried out at any suitable temperature, for example from about -80 to about 120 °C, preferably from about -20 to about 100 °C, more preferably from about -10 to about 80 °C, for a time sufficient to convert the starting compounds into the desired Diels-Alder adduct, such as about 10 minutes to about 6 days, preferably about 3 hours to about 4 days, more preferably about 3 hours to about 2 days. The reaction can be carried out at ambient pressure or under increased pressure. In a preferred embodiment, the reaction is carried out at ambient pressure, such as about 1 bar, or at a pressure of up to 10 bars, preferably up to 5 bars, more preferably up to 2 bars.

According to an embodiment of the invention, the molar ratio of the halofuran of formula (II) to divinylketone is close to stoichiometry (namely 2/1), or above.
The Applicant has found that a molar ratio of the halofuran of formula (II) to divinylketone greater than 2/1 allows for the displacement of the Diels-Alder reaction towards the formation of the cycloadduct of formula (I).

The process according to the invention may comprise a further step of isolation of compound of formula (I). Suitable methods of isolation and purification are known to the person skilled in the art. For example, chromatography techniques, such as liquid chromatography, may be particularly efficient in the process according to the invention.

The present invention also relates to a process for the preparation of a 4,4'-dihalobenzophenone of formula (III): 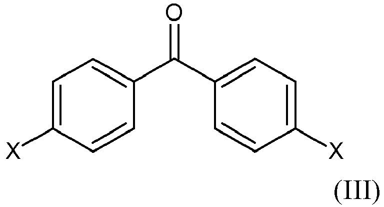 wherein X represents a halogen atom selected from the group consisting of fluorine, chlorine, bromine and iodine, comprising carrying out the dehydration/aromatization of the compound of formula (I) as defined above.
Preferably, X is selected from fluorine and bromine, more preferably fluorine.

The dehydration/aromatization may be represented by the following scheme: 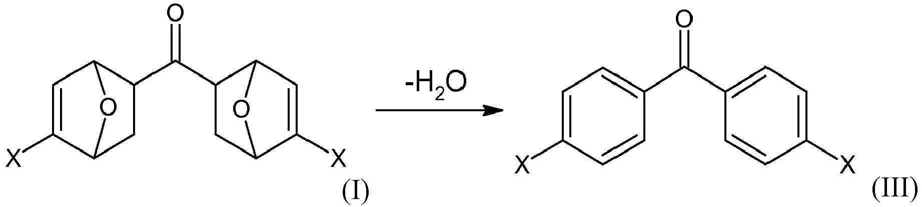 The reaction conditions for aromatization of the compound of formula (I) are known to a person skilled in the art.

According to an embodiment of the invention, the aromatization reaction of the compound of formula (I) requires basic reaction conditions, for example in the presence of an alkoxide compound or hydroxide compound. Suitable alkoxides may be selected from alkali methoxides, such as sodium methoxide and potassium methoxide, preferably sodium methoxide. Suitable hydroxides may be selected from alkali hydroxides, such as sodium hydroxide, potassium hydroxide, lithium hydroxide, cesium hydroxide, preferably sodium hydroxide and potassium hydroxide, more preferably potassium hydroxide.

Suitable solvents for the aromatization reaction may be for example a sulfoxide such as DMSO or an alcohol such as isopropanol.

The aromatization reaction may be carried out at any suitable temperature, for example from about -10 to about 120 °C, preferably from about -5 to about 80 °C, more preferably from about 0 to about 40 °C.

Another object of the invention relates to a process for producing a 4,4'-dihalobenzophenone of formula (III): 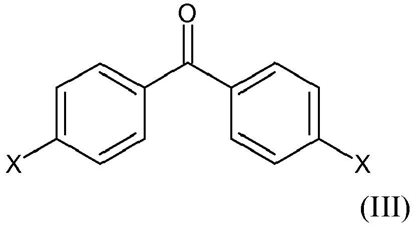 wherein X represents a halogen atom selected from the group consisting of fluorine, chlorine, bromine and iodine,
comprising:
a) providing a compound of formula (I) as defined above,
b) carrying out the dehydration/aromatization of said compound of formula (I).

Alternatively, 4,4'-difluorobenzophenone may be prepared by effecting a halogen-fluorine exchanging reaction between compound of formula (III) wherein X is chlorine, bromine or iodine, and an alkali fluoride, such as potassium fluoride, as described in US 4,453,009.

According to an embodiment of the invention, the compound of formula (I) is obtained by a Diels-Alder reaction between divinylketone and the above-described halofuran of formula (II) according to the process previously detailed.

Once the Diels-Alder reaction is completed, isolation of composition of formula (I) may be carried out before step b).

Alternatively, the mixture obtained at the end of the Diels-Alder reaction and comprising structural isomers of formula (I), (I') and (I") can be directly used in the subsequent step of dehydration/aromatization. In this case, a mixture comprising the following structural isomers is obtained after completing the aromatization:

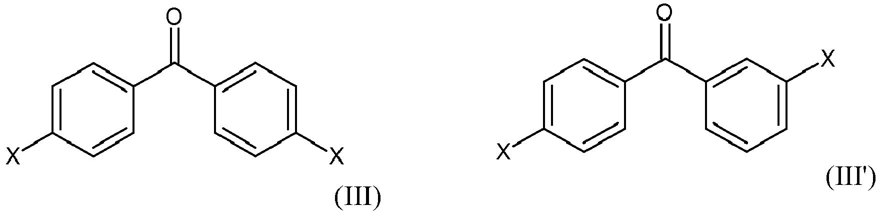

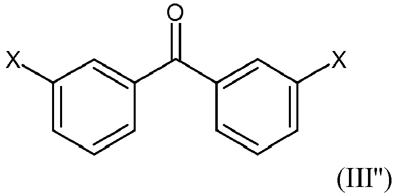

According to an embodiment of the invention, 4,4'-dihalobenzophenone of formula (III) is further isolated by separation methods known to the person skilled in the art. For example, recrystallization may be carried out according to the method described in document US 4,873,372.

Poly(aryl ether ketone)s (PAEK) are a well known class of engineering polymers useful in various fields of scientific and commercial endeavour. PAEK polymers are generally prepared by aromatic nucleophilic substitution of both the halogenides of a 4,4'-dihalobenzophenone (especially, by aromatic nucleophilic substitution of both the fluorides of 4,4'-difluorobenzophenone) by a nucleophilic oxygen atom from a co-monomer. For example, p-hydroquinone, commonly referred to as "hydroquinone" and/or at least one bisphenol (such as bisphenol A, bisphenol S, bisphenol O, 4,4'-dihydroxybiphenyl and mixtures thereof) can be used as the co-monomer; the hydrogen atom from each of the two aromatic hydroxyl groups of p-hydroquinone and/or of the bisphenol is advantageously deprotonated with at least one base (such as NaOH, Na₂CO₃, K₂CO₃ and mixtures thereof) to form a nucleophile which reacts with the 4,4'-dihalobenzophenone (preferably, 4,4'-difluorobenzophenone) to form a PAEK polymer via a nucleophilic substitution mechanism, with the halogen atoms of the 4,4'-dihalobenzophenone acting as leaving groups. Processes for preparing PAEK polymers, including those using a 4,4'-dihalobenzophenone such as 4,4'-difluorobenzophenone, can be found notably in U.S. Pat. Nos. 3,953,400, 3,956,240, 3,928,295, and 4,176,222, all incorporated herein by reference.

Then, facets of the invention relate to :
- the use of a 4,4'-dihalobenzophenone of formula (III) prepared from a compound of formula (I) as previously defined for the manufacture of a poly(aryl ether ketone), in particular poly(ether ether ketone),
- the use of a compound of formula (I) as previously defined for the manufacture of a poly(aryl ether ketone), in particular poly(ether ether ketone), typically with a 4,4'-dihalobenzophenone of formula (III) as synthesis intermediate, and
- the use of a 4,4'-dihalobenzophenone of formula (III) as synthesis intermediate in the manufacture of a poly(aryl ether ketone) [in particular poly(ether ether ketone] from a compound of formula (I) as above defined.

Precisely an object of the present invention concerns a process for the manufacture of a (poly aryl ether ketone), said process comprising the following steps:
i) providing a compound of formula (I) as defined above,
ii) carrying out the dehydration/aromatization of said compound of formula (I) to prepare a 4,4'-dihalobenzophenone of formula (III) as defined above,
iii) optionally, isolating the 4,4'-dihalobenzophenone,
iv) optionally, effecting a halogen-fluorine exchanging reaction between the 4,4'-dihalobenzophenone and an alkali fluoride as explained above ,
v) carrying out the polycondensation of the 4,4'-dihalobenzophenone with at least one aromatic compound comprising 2 hydroxyl groups, in particular with hydroquinone and/or at least one bisphenol, preferably in the presence of at least one base.

The present invention will now be illustrated by the following examples, which are not intended to be limiting.

### EXAMPLES

### a) Diels-Alder reaction of divinylketone with 3-bromofuran

In a Schlenk tube under argon atmosphere, divinyl carbinol (1.0 g, 11.9 mmol) was dissolved in dichloromethane (19 mL), then 2,3-dichloro-5,6-dicyano-1,4-benzoquinone was added (2.94 g, 13.0 mmol). The reaction mixture was stirred at 25°C for 18 hours. The solid part of the reaction media was removed by filtration, and to the filtrate cooled at 0°C were added MgI₂ (496 mg, 1.78 mmol) and 3-bromofuran (8.74 g, 59.5 mmol). The reaction mixture was stirred at 0°C for 18 hours. An NMR analysis of a sample after 3 hours of reaction showed a near complete conversion to the double Diels-Alder adducts. The crude reaction mixture was passed through a short pad of silica gel (elution AcOEt/cyclohexane 50/50). After evaporation of the solvents in vacuo, 4.83 g of a mixture of Diels-Alder adducts (purity 84% in NMR analysis) were obtained.

### b1) Aromatization of the double Diels-Alder adducts

In a carousel tube fitted with a PTFE septum screw cap, Diels-Alder adducts from the example a) (206 mg, 0.46 mmol), dimethyl sulfoxide (1.5 ml) and sodium methoxide solution (25 wt. % in methanol; 41 mg; 0.19 mmol) were charged. The reaction mixture was stirred at room temperature during 2 hours. ¹H NMR (DMSO-d6) show the conversion of the cycloadducts and the formation of dibromobenzophenone (δ 7.78, (d, J= 8.8 Hz), 7.67 (d, J=8.8Hz)). GC analysis calibrated with the commercial product show the formation of 30 mg of 4,4'-dibromobenzophenone and 3 mg of 3,4'-dibromobenzophenone, corresponding to a yield of 21 % for those 2 products.

### b2) Alternative procedure for conducting the aromatization of the double Diels-Alder adducts

In a carousel tube fitted with a PTFE septum screw cap, Diels-Alder adducts from the example a) (202 mg, 0.45 mmol) is weighted. A solution of potassium hydroxide (85%, 39 mg, 0.59 mmol) in isopropanol (1.23 g) was added at 15°C. The reaction mixture was stirred at room temperature during 30 minutes. GC analysis calibrated with the commercial product show the formation of 46 mg of in 4,4'-dibromobenzophenone and 21 mg of 3,4'- dibromobenzophenone, corresponding to a yield of 44 % for those 2 products.

## Claims

1. Compound of formula (I): 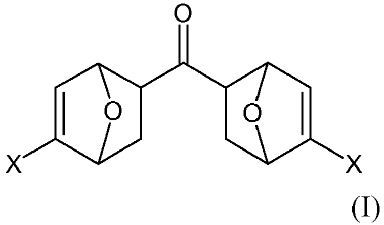 wherein X represents a halogen atom selected from the group consisting of fluorine, chlorine, bromine and iodine.

2. Compound according to claim 1, wherein the halogen atom is selected from bromine and chlorine, preferably chlorine.

3. Process for the preparation of a compound of formula (I) as defined in claim 1 or 2, comprising reacting divinylketone with a halofuran of formula (II): 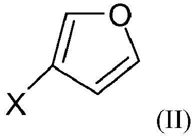 wherein X is as defined above,
and optionally further isolating compound of formula (I).

4. Process according to claim 3, wherein the halofuran is 3-chlorofuran or 3-bromofuran.

5. Process according to claim 3 or 4, wherein the molar ratio of the halofuran of formula (II) to divinylketone is of at least 2/1.

6. Use of a compound of formula (I) as defined in claim 1 or 2, for the preparation of a 4,4'-dihalobenzophenone of formula (III): 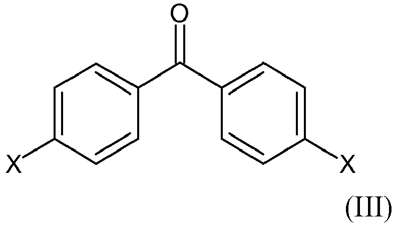 wherein X is as defined above.

7. Process for the preparation of a 4,4'-dihalobenzophenone of formula (III): 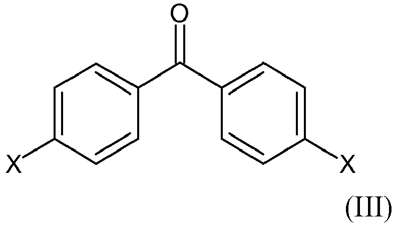 wherein X represents a halogen atom selected from the group consisting of fluorine, chlorine, bromine and iodine,
comprising carrying out the dehydration/aromatization of a compound of formula (I): 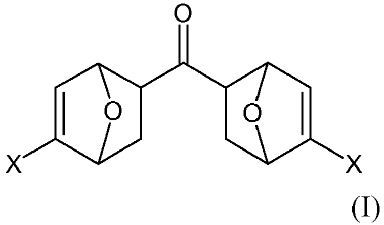 wherein X is as defined above.

8. Process according to claim 7, wherein the dehydration/aromatization is carried out in the presence of an alkali methoxide, preferably sodium methoxide or potassium methoxide, or an alkali hydroxide, preferably sodium hydroxide or potassium hydroxide.

9. Process according to claim 7 or 8, wherein the dehydration/aromatization is carried out in the presence of DMSO or an alcohol.

10. Process for the preparation of a 4,4'-dihalobenzophenone of formula (III): 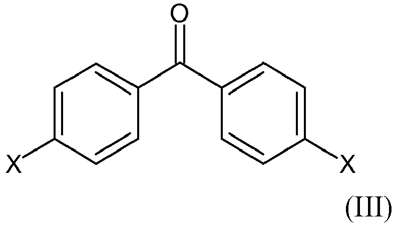 wherein X represents a halogen atom selected from the group consisting of fluorine, chlorine, bromine and iodine,
comprising:
a) providing a compound of formula (I): 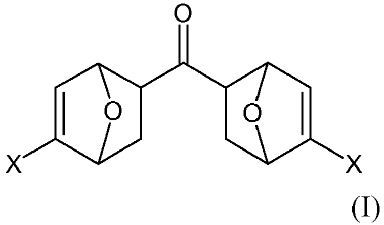 wherein X is as defined above,
b) carrying out the dehydration/aromatization of said compound of formula (I),
c) optionally further isolating compound of formula (III).

11. Process according to claim 10, wherein compound of formula (I) is prepared by reacting divinylketone with a halofuran of formula (II): 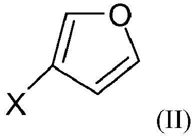 wherein X is as defined above.

12. Use of a compound of formula (I) as defined in claim 1 or 2 for the manufacture of a poly(aryl ether ketone).

13. Use according to claim 12, wherein the poly(aryl ether ketone) is poly(ether ether ketone).

14. Use of a 4,4'-dihalobenzophenone of formula (III) 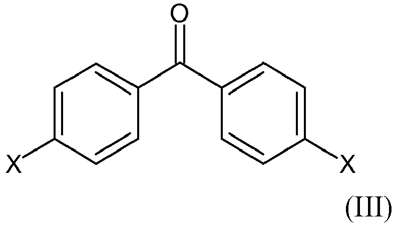 wherein X represents a halogen atom selected from the group consisting of fluorine, chlorine, bromine and iodine, as synthesis intermediate in the manufacture of a poly(aryl ether ketone) from a compound of formula (I) as defined in claim 1 or 2.

15. Use according to claim 14, wherein X represents a fluorine atom.
